## Europäisches Patentamt
## European Patent Office
## Office européen des brevets

(19)

(11) Veröffentlichungsnummer: **0 028 005**
**B1**

(12)
# EUROPÄISCHE PATENTSCHRIFT

(45) Veröffentlichungstag der Patentschrift:
**15.10.86**

(51) Int. Cl.⁴: **A 61 K 7/30**

(21) Anmeldenummer: **80106447.8**

(22) Anmeldetag: **23.10.80**

(54) **Reinigungstablette für Zahnprothesen.**

(30) Priorität: **30.10.79 AT 7005/79**

(43) Veröffentlichungstag der Anmeldung:
**06.05.81 Patentblatt 81/18**

(45) Bekanntmachung des Hinweises auf die Patenterteilung:
**15.10.86 Patentblatt 86/42**

(84) Benannte Vertragsstaaten:
**BE CH DE FR GB LI NL SE**

(56) Entgegenhaltungen:
**DE-A-1 943 441**
**DE-A-2 357 720**
**DE-A-2 527 534**
**FR-A-2 290 187**
**GB-A-1 527 010**

**Die Akte enthält technische Angaben, die nach dem Eingang der Anmeldung eingereicht wurden und die nicht in dieser Patentschrift enthalten sind.**

(73) Patentinhaber: **Gergely, Gerhard, Dr., Gartengasse 8, A-1050 Wien (AT)**

(72) Erfinder: **Gergely, Gerhard, Dr., Gartengasse 8, A-1050 Wien (AT)**

(74) Vertreter: **Büchel, Kurt F., Dr., Patentanwalt Dr. Kurt F. Büchel Bergstrasse 297, FL- 9495 Triesen (LI)**

LIBER, STOCKHOLM 1986

## Beschreibung

Die Erfindung betrifft eine Reinigungstablette für Zahnprothesen enthaltend eine in wässerigem Medium sauer reagierende Komponente und eine alkalisch reagierende Komponente als Bestandteile der Brausemischung und gegebenenfalls weitere Zusätze wie Oxydationsmittel, Aromastoffe, Farbstoffe, Indikatoren, antimikrobielle Mittel, Trägermittel, Trennmittel und/oder proteolytische Enzyme.

Es sind bereits Brausetabletten für die Reinigung von Zahnprothesen bekannt, die im wesentlichen aus Carbonaten und/oder Bicarbonaten, festen organischen Säuren, wie Zitronensäure, Weinsäure und dergleichen, Phosphaten und/oder Polyphosphaten, Monosulfopersäuren, Tensiden, Bindemitteln, waschaktiven Substanzen, antimikrobiellen Mitteln, Farbindikatoren und so weiter bestehen.

Weiterhin sind Mehrschicht-Brausetabletten für die Zahnprothesenreinigung bekannt, bei denen eine zuerst in Lösung gehende, eine kalkbindende organische Säure enthaltende Schicht, eine Grobreinigung oder Vorreinigung der Prothese besorgt, wonach die zweite Schicht, die bereits durch die Wirkung der ersten Schicht angegriffenen Beläge im Zuge einer Fein- oder Nachreinigung entfernen soll.

Bei einem bekannten Reinigungsmittel (FR-A-22 90 187) ist neben der Säure ein durch einen Überzug aus polymerem Material langsamer löslich gemachtes granulatförmigas Neutralisationsmittel enthalten. Das erforderliche Beschichtungsverfahren ist kompliziert. Außerdem wird mit diesem Mittel der Neutralpunkt der Reinigungslösung nicht überschritten, Es kommt also nicht zu einer erwünschten alkalischen Phase mit besonderem Reinigungseffekt.

Ferner sind Reinigungstabletten bekannt (DE-A-25 27 534), bei denen die Beeinträchtigung der Wirkung der Enzyme dadurch behoben wird, daß die Enzyme in einer Schicht enthalten sind, deren Zusammensetzung schneller löslich ist als die den aktiven Sauerstoff liefernde Schicht. Ein Wechsel von einer zunächst sauren über eine neutrale in eine alkalische Phase ist mit dieser bekannten Reinigungstablette nicht erzielbar.

Schließlich ist eine Reinigungstablette bekannt (DE-A-23 57 720), bei der nach einer sauren Phase lediglich eine neutrale Phase erreicht wird und bei der ebenfalls eine erwünschte sich anschließende alkalische Phase fehlt.

Alle bekannten Systeme sind mit einem wesentlichen Nachteil behaftet. Es muß beachtet werden, daß die Verschmutzung einer Prothese grundsätzlich in drei Gruppen eingeteilt werden kann, und zwar:

1. Speisereste fettigen Charakters oder fettigviskosen Charakters (Mehlspeisen, Saucen, Fruchteis)

2. Angelagerte Eiweißstoffe (Fleisch, Michproduktreste)

3. Schwer wasserlösliche mineralische Ablagerungen von Speichel (zahnsteinähnlichen Charakters).

Die meisten Prothesenreinigungsmittel sprechen sehr gut auf die Gruppe 1 an, weniger gut auf die Gruppe 2, fast überhaupt nicht aber auf die Gruppe 3. Der Grund liegt darin, daß mit Tensiden und sauerstoffabspaltenden Substanzen zwar fettige Substanzen abgetragen werden können und auch die durch Verwesungsvorgänge auftretende Geruchsbildung bekämpft werden kann, es jedoch bereits weniger leicht ist, Eiweißstoffe zu entfernen, was nur dann gelingt, wenn sie in die Fettstoffe eingelagert waren und mit diesen weggetragen werden. Kleben diese Eiweißstoffe aber sehr stark an der Prothese und ist die Prothese diesbezüglich sehr stark verschmutzt, versagen die meisten derartigen Mittel. Fast überhaupt unwirksam sind aber alle Mittel in bezug auf die zahnsteinartigen mineralischen Ablagerungen, die nach längerem Gebrauch der Prothese ohne Reinigung eine Gerüstsubstanz bilden, wobei in diesen zahnsteinartigen Ablagerungen Proteine eingelagert sein können.

Die bekannten Prothesenreinigungsmittel versagen vor allem deshalb, weil die Zahnsteinbeläge nur in stark saurem Medium aufgelöst und abgetragen werden können. Die Reinigung von Fetten und das Abtragen durch Tenside ist jedoch vornehmlich im pH von 7 aufwärts sinnvoll, da Tensidreinigungen und vor allem Sauerstoffabspaltugen unter pH 7 technisch nicht realisierbar sind.

Will man nun bei einer Prothesenreinigung alle drei Verschmutzungsursachen bekämpfen und beseitigen, dann sind auch prinzipiell drei Wirkstoffe nötig.

Da es aber nicht möglich ist, ein Reinigungsmittel herzustellen, das zugleich ein saures und ein alkalisches pH hat, ist es erfindungsgemäß notwendig, eine Mehrkomponententablette, in der eine saure und eine alkalische Komponente durch verschiedene Löslichkeit gekennzeichnet sind, zu schaffen, die den Zweck hat, das pH der Reinigungslösung anfangs bestimmte Zeit im sauren Bereich zu halten und in der Folge in den entgegengesetzten alkalischen Bereich zu verschieben, um eine Reinigungskraft auf alle drei Verschmutzungsfaktoren auszuüben.

Grundsätzlich sind hier also zwei Vorgangsweisen möglich: Zum einen kann die Mehrkomponententablette im sauren Bereich beginnen und nach einiger Zeit in den alkalischen Bereich umschlagen. Die andere Möglichkeit ist die Anwendung in der anderen Richtung, nämlich die Alkalinität in den Beginn zu stellen und hernach in den sauren Bereich wechseln.

Die erstgenannte Vorgangsweise ist insofern vorteilhaft, als zu Beginn die zahnsteinartigen mineralischen Ablagerungen, die aufgrund ihrer mikrokristallinen Struktur besonders hartnäckig an der rauhen und zerkratzten oder mechanisch beschädigten Oberfläche der Prothese haften, abgelöst werden und nachher im alkalischen Milieu die leicht beherrschbare Ablösung der Fettschicht erfolgt.

Setzt man nun für das Abtragen der fetten Bestandteile Sulfoperosäuren, Tenside, Polyphosphate, Perborate und ähnliches ein, dann ist es ebenfalls vorteilhaft, wenn diese Komponenten als zweite zur Wirkung kommen. Man kann in vorteilhafter Weise in derselben Zeitspanne, in der diese Alkalinität wirkt, auch Waschmittelenzyme einsetzen, die nach dem Umschlag der Tablette vom sauren in den alkalischen pH-Bereich langdauernd wirksam werden. Die meisten Waschmittelenzyme entfalten ihre Hauptaktivität im Bereich eines pH von 7 bis 9, es sind jedoch auch im sauren Bereich wirkende Waschenzyme bekannt.

Der Erfindung liegt die Aufgabe zugrunde, eine Brausetablette der als bekannt vorausgesetzten Art so auszubilden, daß sie eine besonders gute Reinigungswirkung gegenüber allen drei vorerwähnten Gruppen der Verschmutzungsträger aufweist.

Die Lösung dieser Aufgabe erfolgt dadurch, daß von den beiden Komponenten die alkalisch reagierende Komponente in bezug auf die sauer reagierende Komponente langsamer wasserlöslich ist bzw. später in Lösung geht, um nach Einbringen der Tablette in Wasser zeitweise den pH-Wert unter gleichzeitigem Brausen im sauren Bereich zu halten, und in einem stöchiometrischen Überschuß vorliegt, um nach beendeter Reaktion der Brausemischung den pH-Wert der Lösung in den zum vorhergehend zeitweise aufrechterhaltenen sauren pH-Bereich entgegengesetzten, alkalischen Bereich zu verschieben, wobei ein in der Tablette allfällig zugegenes proteolytisches Enzym in der langsamer wasserlöslichen bzw. später in Lösung gehenden und im stöchiometrischen Überschuß vorliegenden alkalischen Komponente oder in einer von den miteinander reagierenden sauren und alkalischen Komponenten getrennten Schicht enthalten ist.

Vorzugsweise wird die langsamer lösliche bzw. später in Lösung gehende alkalische Komponente durch ihrer Natur nach langsam oder schwer lösliche Salze oder Verbindungen gebildet.

Die langsamer lösliche alkalische Komponente kann verschiedenartig beschaffen sein, sie kann aus an sich langsamer löslichen Verbindungen bestehen, wobei sich "langsamer löslich" auf die saure Komponente bezieht. Ein langsam lösliches Alkali ist beispielsweise wasserfreies Natriumcarbonat. Weiters kann zur Erreichung dieses Zieles zumindest ein Teil der Masse der genannten Komponente durch Schmelzen in größere Schmelzagglomerate und Zerkleinern zur gewünschten Korngröße in einen Zustand übergeführt werden, in welchem sie sich weniger rasch löst, als die andere Komponente. Als langsamer lösliche Komponente können auch Salze oder Verbindungen verwendet werden, die ihrer Natur nach in Wasser schwer löslich bis unlöslich sind, wie zum Beispiel Calciumcarbonat, Calciumhydroxid, Magnesiumoxid, Magnesiumhydroxidcarbonat und dergleichen, welche die durch die vorerwähnten oder in der Folge erläuterten Maßnahmen erzielten Verzögerungen noch unterstützen.

Die langsamere Löslichkeit der alkalischn Komponente kann auch dadurch erzielt oder erhöht werden, daß man die diese Komponente bildenden Stoffe zusammen mit einer eine geringe Lösungsgeschwindigkeit aufweisenden Füllsubstanz, wie zum Beispiel wasserfreies Natriumsulfat, langsam wasserlöslichen polymeren Stoffen und dergleichen verpreßt.

Dabei erfolgt keine Umhüllung der betreffenden Komponente, sondern eine Ausfüllung der Zwischenräume im Preßkörper. Diese Füllmasse muß erst herausgelöst werden, so daß das Lösungswasser wohl ständig, aber nur langsam die alkalisch reagierende Komponente lösen kann. Wäre die genannte Komponente mit einer Überzugsschicht versehen, so würde sich anfangs die andere Komponente ohne wesentliches Schäumen lösen, die mechanische Reinigungswirkung des perlenden Gases ginge also verloren. Sodann wurde nach Losen des Überzuges die alkalische Substanz plötzlich mit der sauren unter momentaner Schäumung reagieren und nicht langsam unter dauerndem Schäumen, wie es mit der erfindungsgemäßen Tablette beabsichtigt ist, bei der von Anfang an auch in der sich langsamer lösenden alkalischen Schicht reaktionsfähige Substanz freiliegt.

Der oben erwähnte Ausdruck "schwer löslich bis unlöslich" entspricht der Definition gemäß DAB 7, wonach eine schwer lösliche Substanz in 100 - 1000 Teilen Lösungsmittel (im gegenständlichen Falle Wasser) löslich ist. Als praktisch unlöslich gelten gemäß DAB 7 Substanzen, die in mehr als 1000 Teilen löslich sind. Als Beispiel praktisch unlöslicher Substanzen seien angeführt das bereits erwähnte Calciumcarbonat mit einem Löslichkeitsprodukt bei 25° C von 4,8 10 $^9$ oder Magnesiumoxid (löslich über das Hyperoxid) von 5,5 x 10$^{-12}$

Unter langsam wasserlöslichen polymeren Stoffen sind solche zu verstehen, die Wasser erst aufnehmen, sodann gegebenenfalls quellen und erst dann langsam in Lösung gehen. Beispiele hierfür sind Kolloide oder Pseudokolloide, die wasserlöslichen Eiweißstoffe, Celluloseäther, Celliloseester, Polyvinylalkohol und dergleichen. Mit besonderem Vorteil werden wegen ihrer leichten Verfügbarkeit Carboxymethylcellulose, Äthylcellulose, Alginsäureester, wie zum Beispiel der Propylester und Gelatine eingesetzt. Die Aufzählung dieser Verbindung schließt jedoch die Verwendung anderer Stoffe mit ähnlichen Eigenschaften wie zum Beispiel von langsam wasserlöslichen Pflanzenfettsäureglyceriden nicht aus.

Wie bereits erwähnt wurde, kann die erfindungsgemäße Reinigungstablette in an sich bekannter Weise auch ein Waschmittelenzym beziehungsweise proteolytisches Enzym enthalten. Dieses Enzym muß jedoch in der langsamer löslichen alkalischen Komponente lebensfähig sein.

Der Zusatz an proteolytischem Enzym beträgt dabei vorzugsweise nicht mehr als 1 Gew.-%.

Das Enzym ist zweckmäßigerweise in einer separaten nicht brausenden Schicht vorhanden.

Die langsamer lösliche bzw. später in Lösung gehende alkalische Komponente ist vorzugsweise ein produkt, welches durch Verpressen eines Gemisches der pulverförmigen, sie bildenden Substanz mit einer langsam wasserlöslichen Substanz erhalten wird.

Weitere Merkmale der Erfindung sind in den weiteren Unteransprüchen beschrieben.

Die erfindungsgemäßen Reinigungstabletten können demnach in Form von Einschichttabletten oder von Mehrschichttabletten vorliegen. Im Falle einer Einschichttablette sind die langsamer lösliche alkalische Komponente und die mit dieser reagierende saure Komponente zusammen mit den gegebenenfalls vorhandenen weiteren Bestandteilen zu einer Tablette verpreßt, wobei beispielsweise 1 - 5 Gew.-% Polyvinylpyrrolidon, bezogen auf die gesamte Tablettenmasse, oder Carbowachs mit einem Molekulargewicht von 4000 bis 20.000 als Bindemittel verwendet werden können.

Bei Mehrschichttabletten liegen die beiden Phasen zum Beispiel in zwei getrennten Schichten vor, wobei noch eine weitere Schicht für das Enzym vorgesehen sein kann. Es ist jedoch auch möglich, das Enzym in einer Zweischichttablette in der Schicht der langsam löslichen alkalischen Komponente vorzusehen.

Eine erfindungsgemäße Zweischichttablette kann z.B. folgendermaßen beschaffen sein:

Die oberste Schicht der Tablette besteht vorzugsweise aus einer Brausemischung mit stöchiometrisch geringerem Carbonatanteil in bezug auf den Säureanteil, die sich mit einem pH von 3 - 5 auflöst und der Zahnsteinentfernung dient. Hier können als Säure ebenfalls neben der Zitronensäure, Fumarsäure, Natriumfumarat, Amidosulfosäure und Betainhydrochlorid eingesetzt werden. Diese Schicht enthält gegebenenfalls einen Indikator und wird in einem gewissen Zeitraum von 5 bis 20 min die mineralischen Gerüstsubstanzen von zahnsteinartigem Charakter angreifen und auflösen.

Die zweite Schicht kann nun aus einem in bezug auf die saure Komponente der ersten Schicht langsamer löslichen Alkali bestehen, das wiederum die konventionellen Produkte wie Kaliumpersulfat, Natriumperborat, Natriumpolysulfat, Kaliumcaroat, Tenside und so weiter enthält. Während die erste Schicht die Lösung ansäuert, werden durch die zweite Schicht die vorher zahnsteinlösenden Säuren neutralisiert und schlägt sodann das pH der Lösung ins Alkalische um, wobei die Tenside sowie die Perborate und Persulfate wirksam werden.

Zum Zwecke der Stabilitätserhöhung können diese Schichten getrennt sein, so daß beispielsweise die alkaliempfindlichen Persulfate und Perborate in einer neutralen Drittschicht eingehüllt sind, während die mittlere Schicht lediglich dazu dient, ein sehr starkes Alkali vom Typ des Polyphosphates langsam zur Alkalisierung abzugeben.

Selbstverständlich können in einer dieser beiden Schichten oder in einer getrennten dritten Schicht proteolytische Enzyme untergebracht werden.

Vorzugsweise bestehen, wie erwähnt, die erfindungsgemäßen Reinigungstabletten aus drei Schichten, weil bei der Zweischichttablette die Wirkung sehr davon ahhängt, welche Phase beziehungsweise Schicht, wenn die Tablette in den mit Wasser gefüllten, der Prothesenbehandlung dienenden Becher eingebracht wird, auf dem Boden des Bechers zu liegen kommt. Bei einer Dreischichttablette hat die dritte und äußere, das Enzym tragende Schicht unter anderem die Aufgabe, für das Funktionieren des Systems zu sorgen, wenn die Tablette so in das Wasser fällt, daß beispielsweise die saure Schicht, deren Lösung zuerst beabsichtigt ist, flach auf dem Boden des Bechers liegt. In diesem Falle wird nämlich die dritte, das Enzym tragende Schicht zu oberst liegen und aufgrund ihrer Natur und ihrer sehr schweren Löslichkeit verhindern, daß eine zu rasche Lösung der zweiten, wenn auch ebenfalls in bezug auf die saure Schicht langsamer löslichen alkalischen Schicht erfolgt und dadurch die gewünschte Reinigungswirkung der sauren Phase verkürzt wird. Dies wäre bei der Zweischichttablette der Fall, da zwar bei auf dem Boden liegenden saurer Schicht, die langsamer lösliche alkalische Schicht die gewünschte lange Zeit zu ihrer Lösung braucht, aber auch die saure Schicht verlangsamt in Lösung geht. Es ist somit der ganze Prozeß zuungunsten einer langdauernden stark sauren Anfangsphase verschoben.

Die erfindungsgemäßen Brausetabletten besitzen den weiteren Vorteil, daß, wenn als Tablettenbestandteil ein Caroat zugegen ist, nach der Neutralisierung der sauren Komponente und dem Umschlag in den deutlich alkalischen Bereich der Behandlungslösung der Brauseeffekt verlängert werden kann, weil das Caroat auf und mit dem Alkali unter Sauerstoffabgabe zerfällt. Die langsamer lösliche Komponente enthält daher vorzugsweise ein langsam lösliches Alkali wie zum Beispiel wasserfreies Natriumcarbonat, und Caroat. Das wasserfreie Natriumcarbonat benötigt bekanntlich gewisse Zeit, um Wasser aufzunehmen und sich in das wasserhaltige Salz zu verwandeln und in Lösung zu gehen. Innerhalb dieser Zeit findet kaum eine Reaktion des in dieser Schicht vorliegenden Kaliumcaroats statt, so daß allein durch das physikalische Moment der Lösungsvorgang der zweiten Schicht verlangsamt wird. Ab pH 7, also nach Neutralisation der sauren Phase, erfolgt praktisch nur mehr eine Auflösung des Carbonats und ein Zerfall des Caroats unter Sauerstoffentwicklung. Das in der dritten Schicht vorhandene Enzym kann vor der insbesondere in der mittleren zweiten Schicht ziemlich lang andauernden Sauerstoffwirkung geschützt werden, indem sie in ein langsam lösliches neutrales Salz, wie Natriumsulfat, eingebettet wird, wobei diese Schicht mit quellfähigen Stoffen der obengenannten Art versehen ist.

Mit einem derartigen System ist es also möglich, nicht nur eine langdauernde saure und eine abschließende alkalische Reinigungsperiode zu erzielen, sondern eine Sauerstoffdurchperlung zu erzielen, die vom sauren Bereich von pH 2 bis in den alkalischen Bereich bis pH 8,5 reicht. Es ist klar, daß eine langdauernde geringfügige Sättigung der Lösung mit Sauerstoff wesentlich wirksamer ist, als eine momentane rasche Sauerstoffabgabe.

Es ist für den Fachmann offensichtlich, daß im Rahmen der Erfindung zur Erzielung des gewünschten Effektes und zur Realisierung des der Erfindung zugrundeliegenden Gedankens die Komponenten der Reinigungstablette auf verschiedenartigste Weise kombiniert werden können. Wesentlich ist dabei nur, daß die alkalisch reagierende Verbindung der alkalischen Komponente der Tablette derart langsamer löslich ist, daß die Lösung vorübergehend entsprechend deutlich sauer reagiert, um sodann in den entgegengesetzten alkalischen pH-Bereich zu wechseln. Es ist offensichtlich, daß zu diesem Zweck die langsamer lösliche alkalische Komponente in einem stöchiometrischen Überschuß vorhanden sein muß.

Vorzugsweise enthält die Tablette einen zur Kenntlichmachung des pH-Wechsels vom sauren in den alkalischen Bereich befähigten Indikatorfarbstoff.

Das pH der Lösung liegt anfangs vorzugsweise unter 6,5.

Die Erfindung wird anhand der nachfolgenden Beispiele näher erläutert.

In Beispiel 1 wird die Herstellung einer langsam löslichen Alkalikomponente beschrieben:

**Beispiel 1**

20 Gew.-Teile wasserfreies Natriumcarbonat werden mit 5 Gew.-Teilen Magnesiumhydroxidcarbonat vermischt und mit 1 Gew.-Teil Polyvinylalkohol, gelöst in 3 Gew.- Teilen Methylenchlorid granuliert. Die langsamere Löslichkeit der alkalischen Komponente ist darauf zurückzuführen, daß die gleichzeitige Anwesenheit des hochalkalischen aber schwer löslichen Magnesiumhydroxidcarbonats die Reaktionsfähigkeit von Natriumcarbonat gegenüber Säuren in jedem gewünschten Maße herabsetzt.

Das folgende Beispiel beschreibt die verzögerte Alkaliabgabe in Tablettengemischen:

**Beispiel 2**

a) Typische Mischungen für anfangs saure, dann später alkalische Reaktion: 10 Gew.-Teile Natriumbicarbonat, 30 Gew.-Teile Kaliumcaroat, 5 Gew.-Teile Sulfamidsäure und 10 Gew.-Teile Zitronensäure.

In diesem Falle empfiehlt sich besonders der Zusatz von 2 - 4 Gew.-Teile Cetylammoniumbromid.

b) 60 Gew.-Teile dieser Mischung werden mit 50 Gew.-Teilen der langsam löslichen Alkalikomponente gemäß Beispiel 1 versetzt und zu Tabletten gepreßt. Durch geeignete Mischungen, verschiedenster Komponenten erhält man pH-Umschlagverzögerungen von 1 - 3 Stunden.

Die folgenden Beispiele beschreiben Mehrschichttabletten:

Bei Mehrschichttabletten kann man die oben erwähnten Verhältnisse dadurch vereinfachen, daß man die beiden Phasen in eine Zweischicht- oder noch vorteilhafter in eine Dreischichttablette bringt.

Die pH-Verhältnisse lassen sich in diesem Fall durch Dosierung der einzelnen Schichten elegant regeln und man kann sowohl in der Wahl der Dosierung als auch in der Wahl der entsprechenden Komponenten jede gewünschte Verzögerung im pH-Umschlag bei jedem gewünschten pH erzielen.

Tabletten bei denen die Alkali abgebende Schicht verzögert löslich ist, können hergestellt werden, indem man die Mischung gemäß Beispiel 2 a) und sodann die Mischung gemäß Beispiel 1 zu einer Tablette verpreßt.

**Beispiel 3**

In die herabgezogene Matrize einer Tablettenpresse werden 1,5 g einer Mischung bestehend aus 75 Gew.-Teilen Amidosulfonsäure, 10 Gew.-Teilen Natriumcarbonat, 15 Gew.-Teilen Natriumcarbonat, 12 Gew.-Teilen Kaliumcaroat, 2 Gew.-Teilen Polyäthylenglycol (Molekulargewicht 6000) und 2 Teilen waschaktiver Sustanz, wie Alkylarylsulfonate, Fettsäurekondensationsprodukte und dergleichen eingebracht. Diese erste ansäuernde Schicht einer Dreischichttablette wird bei einem Druck von 0,5 - 1 kbar vorverdichtet.

Auf diese Schicht werden sodann 1,5 g einer Mischung aus 10 Gew.-Teilen Natriumperborat, 60 Gew.-Teilen Natriumcarbonat, 1 Gew.-Teil waschaktiver Substanz (wie oben), 6 Gew.-Teilen Natriumpyrophosphat, 50 Gew.-Teilen Kaliumcaroat und 10 Gew.-Teilen eines Fettsäurepartialglycerids aus natürlichen geradkettigen Pflanzenfettsäuren mit einer Kettenlänge von $C_8$ - $C_{12}$ aufgebracht und ebenfalls bei einem Druck von 0,5 bis 1 kbar vorverdichtet.

Als dritte Schicht werden auf diese beiden Schichten 0,5 g eines Gemisches aus 40 Gew.-Teilen wasserfreiem Natriumsulfat, 3 Gew.-Teilen Polyvinylpyrrolidon, 0,1 Gew.-Teil Ascorbinsäure, 4 Gew.-Teile Magnesiumstearat und 1 Gew.-Teil proteolytisches Enzym aufgebracht. Nach dem Einbringen dieser Schicht in die Matrize wird bei 8 kbar zur fertigen Dreischichttablette verpreßt.

Beim Einbringen dieser Tablette in lauwarmes Wasser geschieht folgendes:

Die erste saure Schichte beginnt unmittelbar zu brausen und stellt das Wasser nach 10 - 20 s auf ein pH von 2 - 3 ein. Zugleich zieht die dritte enzymhaltige Schichte Wasser an, wobei das wasserfreie Natriumsulfat Wasser aufnimmt und durch den hochviskosen Polyvinylalkohol sozusagen verstopft wird. Dadurch wird die mittlere alkalische Schichte unbeschadet der Lage der Tablette (1. Schichte oben oder unten) weitgehend von Auflösung und Reaktion geschützt. .

Erst nach 10 - 15 min, wenn die saure erste Schichte aufgelöst ist, beginnt die zweite alkalische Schichte zu reagieren und verschiebt das pH über 7 (ca. 20 min) bis in den Bereich von 8 - 8,5/ 30 min). Nach etwa 1 Stunde beginnt sich die sehr stark verzögerte dritte Schichte zu lösen und gibt die Enzyme frei, wobei etwa noch vorhandene Spuren aktiven Sauerstoffes durch die Ascorbinsäure zerstört werden.

**Patentansprüche**

1. Reinigungstablette für Zahnprothesen enthaltend eine in wässerigem Medium sauer reagierende Komponente und eine alkalisch reagierende Komponente als Bestandteile der Brausemischung und gegebenenfalls weitere Zusätze wie Oxydationsmittel, Aromastoffe, Farbstoffe, Indikatoren, antimikrobielle Mittel, Trägermittel, Trennmittel und/oder proteolytische Enzyme,
dadurch gekennzeichnet,
daß von den beiden Komponenten die alkalisch reagierende Komponente in bezug auf die sauer reagierende Komponente langsamer wasserlöslich ist bzw. später in Lösung geht, um nach Einbringen der Tablette in Wasser zeitweise den pH-Wert unter gleichzeitigem Brausen im sauren Bereich zu halten, und in einem stöchiometrischen Überschuß vorliegt, um nach beendeter Reaktion der Brausemischung den pH-Wert der Lösung in den zum vorhergehend zeitweise aufrechterhaltenen sauren pH-Bereich entgegengesetzten, alkalischen Bereich zu verschieben, wobei ein in der Tablette allfällig zugegenes proteolytisches Enzym in der langsamer wasserlöslichen bzw. später in Lösung gehenden und im stöchiometrischen Überschuß vorliegenden alkalischen Komponente oder in einer von den miteinander reagierenden sauren und alkalischen Komponenten getrennten Schicht enthalten ist.

2. Reinigungstablette nach Anspruch 1,
dadurch gekennzeichnet,
daß die langsamer lösliche bzw. später in Lösung gehende alkalisch reagierende Komponente durch ihrer Natur nach langsam oder schwer lösliche Salze oder Verbindungen gebildet ist.

3. Reinigungstablette nach Anspruch 1,
dadurch gekennzeichnet,
daß die langsamer lösliche bzw. später in Lösung gehen alkalisch reagierende Komponente ein Produkt, erhalten durch Verpressen eines Gemisches der pulverförmigen, sie bildenden Substanz mit einer langsam wasserlöslichen Substanz ist.

4. Reinigungstablette nach einem der Ansprüche 1 bis 3,
dadurch gekennzeichnet,
daß die ansäuernde und die alkalisierende Komponente in zwei getrennten Schichten und das Enzym in einer dritten Schicht vorliegt.

5. Reinigungstablette nach einem der Ansprüche 1 bis 4,
dadurch gekennzeichnet,
daß die Tablette wenigstens einen zur Kenntlichmachung des pH-Wechsels vom sauren in den alkalischen Bereich oder umgekehrt befähigten Indikator enthält.

**Claims**

1. Cleaning tablet for dental plates containing a component that reacts acidic in aqueous solutions and a component that reacts alkaline as a component part of the effervescent mixture, as well as eventually further additives such as oxidants, aromatics, coloring substances, indicators, antimicrobial agents, carrier substances, separating agents and/or proteolytic enzymes
wherein
of the two components the component that reacts alkaline relative to the component that reacts acidic is soluble in water at a slower rate or is dissolved at a later time in order to keep the pH value in the acidic range at times during the effervescence after the tablet has been put into the water, said component that reacts alkaline also being present in stoichiometric excess in order after the completed reaction of the effervescent mixture to shift the pH value of the solution into the opposing alkaline range that was maintained beforehand at times, a proteolytic enzyme eventually present in the tablet being contained in the alkaline component that is soluble in water at a slower rate or dissolving at a later time, and that is present in stoichoimetric excess or in a layer separated by the acidic and alkaline components that react with one another.

2. Cleaning tablet as claimed in claim 1,
wherein
the alkaline-reacting component that is soluble in water at a slower rate or that dissolves at a later time is formed by salts or compounds that by virtue of their nature dissolve slowly or sparingly.

3. Cleaning tablet as claimed in claim 1, wherein the alkaline-reacting component that is soluble at a slower rate or that dissolves at a later time is a product, obtained by pressing a mixture of the powdery substance of which it is comprised, is a product with a substance that is soluble in water at a slow rate.

4. Cleaning tablet as claimed in any one of claims 1 to 3, wherein the acidifying and ths alkalizing components are present in two separats layers, and the enzyme in a third layer.

5. Cleaning tablet as claimed in any one of claims 1 to 4, wherein the tablet contains at least one indicator that is capable of indicating the change of pH from the acidic into the alkaline range or vice versa.

## Revendications

1. Comprimé pour le nettoyage de prothèses dentaires, contenant un composant à réaction acide et un composant à réaction alcaline dans l'eau comme constituants du mélange effervescent et éventuellement d'autres additifs comme un oxydant, des substances d'aromatisation, des colorants, des indicateurs, des agents antimicrobiens, des supports, des agents de séparation et/ou des enzymes pratéolytiques, comprimé caractérisé en ce que, des deux composants, le composant à réaction alcaline est plus lentement soluble dans l'eau que le composant à réaction acide ou passe plus tard en solution, de façon qu'après introduction du comprimé dans l'eau le pH demeure temporairement dans le domaine acide avec effervescence simultanée et que ce composant alcalin est présent en un excès stoéchiométrique de façon qu'après achévement de la réaction du mélange effervescent le pH de la solution vire dans le domaine alcalin, opposé au domaine acide précédemment maintenu temporairement, une enzyme protéolytique contenue éventuellement dans le comprimé étant contenue dans le composant alcalin présent en excès et qui se dissout plus lentement dans l'eau ou passe plus tard en solution ou est présente dans une couche séparée des composants alcalin et acide à réaction mutuelle.

2. Comprimé de nettoyage selon la revendication 1, caractérisé en ce que le composant à réaction alcaline, plus lentement soluble ou passant plus tard en solution, est formé par nature de sels ou composés solubles lentement ou difficilement.

3. Comprimé de nettoyage selon la revendication 1, caractérisé en ce que le composant à réaction alcaline, lentement soluble ou passant plus tard en solution, est un produit obtenu par compression d'un mélange pulvérulent qui le forme avec une substance lentement soluble dans l'eau.

4. Comprimé de nettoyage selon l'une des revendications 1 à 3, caractérisé en ce que le composant d'acidification et le composant d'alcalinisation sont contenus dans deux couches séparées et l'enzyme est présente dans une troisième couche.

5. Comprimé de nettoyage selon l'une des revendications 1 à 4, caractérisé en ce que le comprimé contient au moins un indicateur capable de faire connaître le virage de pH du domaine acide au domaine alcalin ou inversement.